# EUROPEAN PATENT SPECIFICATION

(11) **EP 0 457 295 B1**
(45) Date of publication and mention of the grant of the patent: **09.04.1997**
(21) Application number: 91107844.2
(22) Date of filing: 15.05.1991
(51) Int. Cl.: A61K 31/00

(54) **Use of a modulator of protein phosphorylation in the treatment of amyloidosis associated with Alzheimer's disease**
Verwendung eines Modulators der Proteinphosphorylierung zur Behandlung der Amyloidosis in Zusammenhang mit der Alzheimerschen Krankheit
Utilisation d'un modulateur de la phosphorylation des protéines comme médicament dans le traitement de l'amyloidose associé à la maladie d'Alzheimer

(30) Priority: 16.05.1990 US 524202
(43) Date of publication of application: 21.11.1991
(73) Proprietor: THE ROCKEFELLER UNIVERSITY, New York, NY 10021 (US)
(72) Inventor: Buxbaum, Joseph D., Flushing, N.Y. 11354 (US); Gandy, Samuel E., New York, N.Y. 10128 (US); Greengard, Paul, New York, N.Y. 10021 (US)
(74) Representative: VOSSIUS & PARTNER

(56) References cited:
- EP-A- 0 215 171
- PROCEEDINGS OF THE NATIONAL ACADEMY OF SCIENCES USA, vol. 87, no. 15, 6th August 1990, pages 6003-6006; J.D. BUXBAUM et al.: "Processing of Alzheimer beta/A4 amyloid precursor protein: Modulation by agents that reglate protein phosphorylation"
- PROCEEDNGS OF THE NATIONAL ACADEMY OF SCIENCES USA, vol. 86, no. 19, October 1989, pages 7606-7610; D. GOLDGABER et al.: "Interleukin 1 regulates synthesis of amyloid beta-protein precursor mRNA in human endothelial cells"
- JOURNAL OF NEUROPATHOLOGY AND EXPERIMENTAL NEUROLOGY, vol. 29, no. 3, 1970, pages 463-478; A.B. JOHNSON: "Nucleoside phoshatase activities associated with the tangles and plaques of Alzheimer's disease: A histochemical study of natural and experimenatl neurofibrillary tangles"
- ANNUALS OF THE NEW YORK ACADEMY OF SCIENCES, vol. 568, 1989, pages 198-208; R. A. NIXON: "Calcium-activated neutral proteinases as regulators of cellular function"
- PROG. CLIN. BIOL. RES., vol. 317, 1989, pages 769-780; T. SAITOH et al.: "Aberrant protein phosphorylation and cytoarchitecture in Alzheimer's disease"
- EXPERIMENTAL NEUROLOGY, vol. 112, no. 1, April 1991, pages 95-103; M.P. MATTSON: "Evidence for the involvement of protein kinase C in neurodegenerative changes in cultured human cortical neurons"

## Description

The present invention relates to the use of at least one agent that modulates or effects the phosphorylation of proteins in mammalian cells for the production of a pharmaceutical composition for the treatment of amyloidosis associated with Alzheimer's disease.

Alzheimer's disease (AD) is a brain disorder characterized by abnormal protein phosphorylation and altered protein catabolism. From the work of several laboratories, altered protein phosphorylation has been implicated in the formation of the intracellular neurofibrillary tangled found in Alzheimer's disease. However, a role for protein phosphorylation in the catabolism of the β/A4 protein precursor (βAPP) has not been demonstrated.

Alzheimer's disease is the most common single cause of dementia in late life. Individuals with Alzheimer's disease are characterized by progressive memory impairments, loss of language and visuospatial skills and behavior deficits. The cognitive impairment of individuals with Alzheimer's disease is the result of degeneration of neuronal cells located in the cerebral cortex, hippocampus, basal forebrain and other brain regions. Histologic analyses of Alzheimer's disease brains obtained at autopsy demonstrated the presence of neurofibrillary tangles (NFT) in perikarya and axons of degenerating neurons, extracellular neuritic (senile) plaques, and amyloid plaques inside and around some blood vessels of affected brain regions. Neuritic plaques are located at degenerating nerve terminals (both axonal and dendritic), and contain a core compound of amyloid protein fibers. Cerebrovascular amyloid protein material is found in blood vessels in the meninges and the cerebral cortex (Glenner and Wong, 1984, Biochem. Biophys. Res. Commun., 120:885-890; Glenner and Wong, 1984, Biochem. Biophys. Res. Commun., 122:1131-1135; Wong et al, 1985, Proc. Natl. Acad. Sci., U.S.A., 82:8729-8732).

A central feature of the pathology of Alzheimer's disease is the deposition of amyloid protein within plaques. The 4 kDa amyloid protein (also referred to as A4, APC, β-amyloid or BAP) is a truncated form of the larger amyloid precursor protein (βAPP) which is encoded by a gene localized on chromosome 21 (Goldgaber et al, 1987, Science, 235:877-880; Kang et al, 1987, Nature, 325:733-736; Jenkins et al, 1988, Biochem. Biophys. Res. Commun., 151:1-8; Tanzi et al, 1987, Science, 235:880-885).

In summary, Alzheimer's disease is characterized by certain neuropathological features including intracellular neurofibrillary tangles, primarily composed of cytoskeletal proteins, and extracellular parenchymal and cerebrovascular amyloid (Katzman, R., Biological Aspects of Alzheimer's Disease, Cold Spring Harbor, New York). Biochemically, Alzheimer's disease is characterized by changes in protein phosphorylation. Two components of neurofibrillary tangles, namely the microtubule-associated protein tau and neurofilament proteins found in the neurofibrillary tangles are abnormally phosphorylated (perhaps by calcium/calmodulin-dependent protein kinase II (CaMKII)) (Sternberger, N.H., Sternberger, L.A. and Ulrich, J., (1985), Proc. Natl. Acad. Sci., U.S.A., 82:4274 and Flament, S. and Delacourte, A., (1989), FEBS Lett., 247:213). Protein kinase C (PKC) activity is reported to be reduced in Alzheimer's disease brain as well as in fibroblasts from Alzheimer's disease, familial Alzheimer's disease and Down's syndrome patients (Cole, G., Dobkins, K.R., Hausen, L.A., Terry, R.D. and Saitoh, T., (1988), Brain Res., 452:165-174 and Huyhn, T.V., Cole, G., Katzman, R., Haung, R.-P. and Saitoh, T. (1989), Arch. Neurol., 46:1195-1199). A cytosolic 60 kDa phosphoprotein is reported to be present at two-fold higher concentration in Alzheimer's disease brain when compared to controls (Saitoh, T. and Dobkins, K.R. (1986), Proc. Natl. Acad. Sci., 83:9764-9767).

The major constituent of cerebral plaques and cerebrovascular amyloid, the β/A4 protein, is derived from a large transmembrane protein, β/A4 amyloid precursor protein, or βAPP. βAPP is comprised of three major isoforms, βAPP₆₉₅ βAPP₇₅₁ and βAPP₇₇₀, which arise from alternate splicing and are denoted by the lengths of their primary sequences. This protein is a phosphoprotein, and it has previously been proposed that the altered phosphorylation associated with Alzheimer's disease might affect not only tangle proteins, but might also alter the metabolism of βAPP, perhaps leading to deposition of βA4 (Gandy et al, (1988), Proc. Natl. Acad. Sci., U.S.A. 85:6218).

Using a synthetic peptide corresponding to a potential phosphorylation site in the βAPP transmembrane and cytoplasmic domain, it has previously been shown that this peptide is a highly efficient substrate for PKC or CaM K II. The putative PKC phosphorylation site of βAPP, located seven residues from the predicted transmembrane/cytoplasmic border of the protein, is similar to a PKC-phosphorylation site found in both the epidermal growth factor receptor and in the interleukin-2 receptor. Phosphorylation of either of these two receptors by PKC at these sites targets them for internalization. By analogy, phosphorylation of βAPP may regulate its internalization and catabolism.

Applicants have extended the above observations by studying the effects of agents that regulate protein phosphorylation on the metabolism of βAPP, using PC12 rat pheochromocytoma cells as a model system. The metabolism of βAPP in PC12 has been well characterized (Wiedemann, A., Konig, G., Buke, D., Fischer, P., Salbaum, J.M., Masters, C.L. and Beyreuther, K. (1989), Cell, 57:115) making this cell line particularly useful. Applicants observed that βAPP metabolism is regulated by agents that regulate protein phosphorylation and it is believed that alterations in protein phosphorylation will modify the deposition of β-amyloid in extracellular sites characteristic of Alzheimer's disease.

The present invention concerns the use of a kinase modulator or a phosphatase modulator regulating protein phosphorylation of proteins found in intracellular neurofibrillary tangles and extracellular amyloid plaques, the modulator increasing or decreasing the rate of proteolytic processing of such proteins, for the preparation of a pharmaceutical composition for the treatment of amyloidosis associated with Alzheimer's disease in a mammalian patient or for inhibiting production of Alzheimer type amyloidosis in a mammal wherein said kinase or phosphatase modulator is at least one activator or inhibitor of protein kinase or phosphatase, the modulator altering the proteolytic processing of proteins found in intracellular neurofibrillary tangles and extracellular amyloid plaques.

The present invention also relates to an in vitro method for screening for agents that modulate amyloid formation comprising contacting mammalian cells with an agent suspected of being capable of modulating the phosphorylation proteins and detecting for alterations in the degradation of the amyloid precursor protein comprising the steps of:
(a) providing mammalian cells or tissue sections in culture;
(b) radioactively labeling proteins produced by the mammalian cells during anabolism; then
(c) allowing the mammalian cells to continue metabolizing in a suitable, label-free media;
(d) contacting the mammalian cells at the start of or during step (c) with an agent suspected of being capable of modulating phosphorylation of proteins that occurs during cell metabolism;
(e) lysing the mammlian cells;
(f) immunoprecipitating the labeled βAPP fragments moieties with an antibody against βAPP; and
(g) comparing the immunoprecipitated βAPP or βAPP fragments to standard βAPP or βAPP fragments to detect changes in APP degradation and β-amyloid production.

The present invention also is directed to the use of an agent that modulates phosphorylation of proteins for the preparation of a diagnostic composition for screening for agents that modulate amyloid formation in a normal or transgenic whole animal comprising administering to said animal an agent suspected of being capable of modulating phosphorylation of proteins and detecting neurodegenerative changes in the brain of the animal.

### BRIEF DESCRIPTION OF THE DRAWINGS

Fig. 1 are autoradiographs of PAGE gels from immunoprecipitates of [³⁵S]methionine labeled PC12 cells.

Fig. 2 are three graphs showing the effect of agents that modulate PKC activity on βAPP proteins in PC12 cells.

Fig. 3 are three graphs showing the effect of PDBu on mature βAPP proteins.

Fig. 4 are three graphs showing the regulation of βAPP processing by okadaic acid as a function of time.

Fig. 5 are three graphs showing the regulation by okadaic acid of processing mature βAPP as a function of time.

### DETAILED DESCRIPTION OF THE DRAWINGS

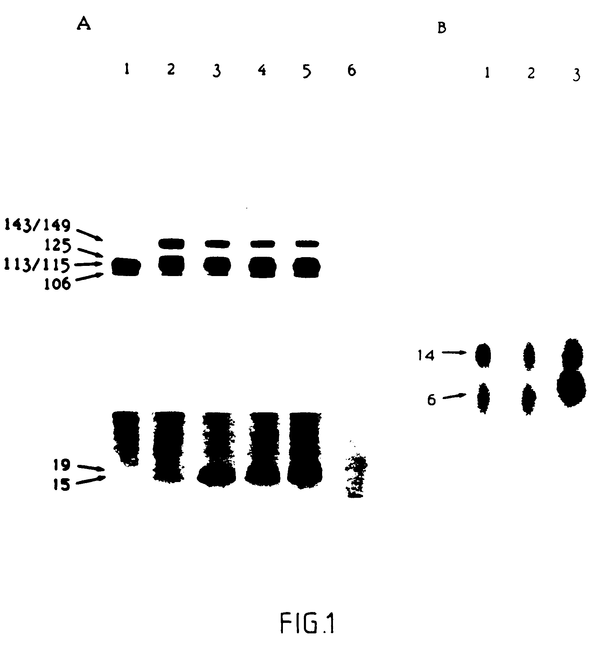

### Figure 1. Regulation of βAPP processing by PDBu and okadaic acid

(A) Identification of βAPP and βAPP -derived peptides in PC12 cells by immunoprecipitation with anti-βAPP⁶⁴⁵⁻⁶⁹⁴ antibodies. Autoradiographs of PAGE gels from immunoprecipitates of [³⁵S]methionine labeled PC12 cells are shown. In the upper panel an autoradiograph of the higher molecular weight region of a 6-18% gradient gel is shown. In the lower panel an autoradiograph of a longer exposure of the lower molecular weight of the gel including the 15 kDa and 19 kDa peptides is shown.
(B) Lanes: 1, Immunoprecipitate of labeled PC12 cells under control conditions at zero-time; 2-6, Immunoprecipitates of labeled PC12 cells at 45 minutes of chase with additions at zero time; 2, Control cells; 3, Cells treated with 1µM PDBu; 4, Cells treated with 1µM okadaic acid; 5, Cells treated with 1µM PDBu and 1µM okadaic acid; 6, Cells treated with 1µM PDBu and 1µM okadaic acid immunoprecipitated in the presence of 100 µM βAPP⁶⁴⁵⁻⁶⁹⁴ peptide. The putative βAPP forms in PC12 cells are related to human βAPP₆₉₅ as determined by limited proteolysis. The 113/115 kDa doublet (lane 1) and the 143/149 kDa doublet (lane 2) were excised from the gel shown in A and subjected to limited proteolysis digestion with V8 protease and compared with similarly treated in vitro transcribed and translated human βAPP₆₉₅ cDNA (lane 3).

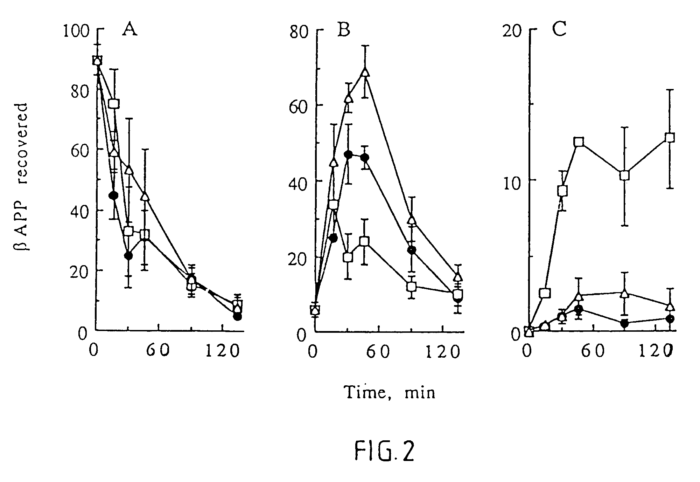

### Figure 2. Regulation of βAPP processing by PDBu and H-7 as a function of time

PC12 cells were metabolically labeled for 20 minutes with [³⁵S] methionine followed by a chase period with complete medium for the indicated times. Agents regulating PKC activity were added at the start of the chase period (zero-time). Following cell lysis, extracts were subjected to immunoprecipitation, NaDodSO₄-PAGE and autoradiography. The various forms of βAPP were quantified by scanning the autoradiogram. The results shown are the means ± SEM of four experiments. The data were normalized such that the total βAPP_{751/770} recovered (i.e., immature and mature) at zero-time was taken as 100 units. The levels of the COOH-terminal βAPP fragments were insignificant at zero-time. In A and B, recovery is expressed at percent of total at zero-time. In C, recovery is expressed as arbitrary units. (A) Immature βAPP_{751/770}. (B) Mature βAPP_{751/770}. (C) 15 kDa COOH-terminal fragment. Control cells(-●-), PDBu-treated cells(-□-), H-7-treated cells(-△-).

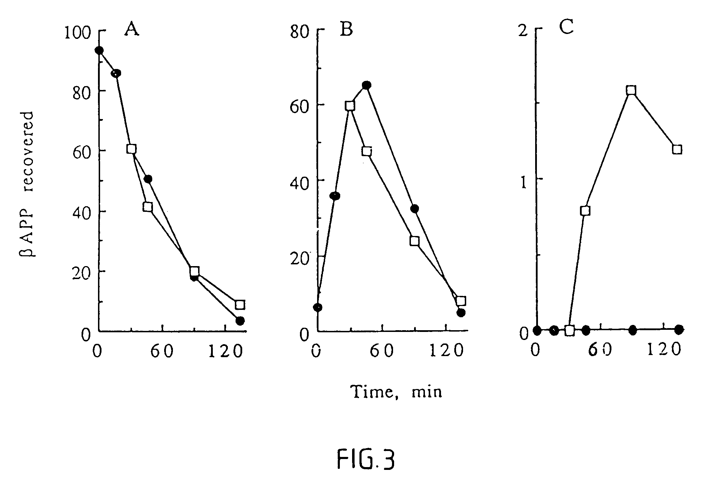

### Figure 3. Regulation by PDBu of processing of mature βAPP as a function of time

PDBu was added at 35 minutes of chase to allow the mature βAPP to approach maximal levels. All other experimental details were as described in the legend to Fig. 2. The experiment shown is representative of three separate experiments. Experimental points are the means of duplicate determination.
(A) Immature βAPP_{751/770}. (B) Mature βAPP_{751/770}. (C) 15 kDa COOH-terminal fragment. Control cells(-●-), PDBU-treated cells(-□-).

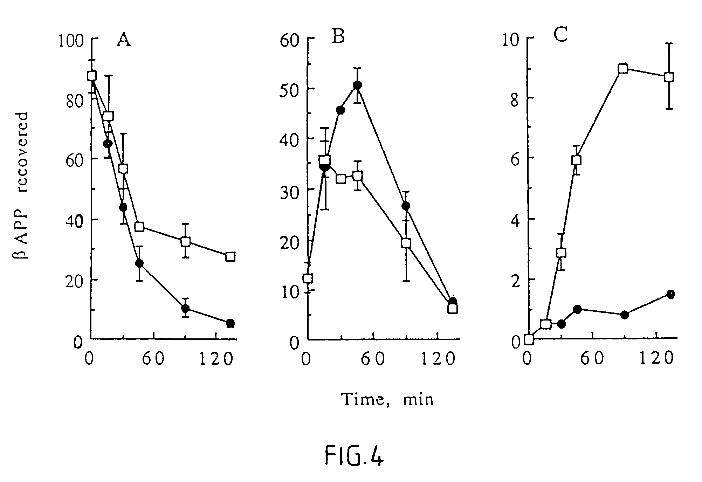

### Figure 4. Regulation of βAPP processing by okadaic acid as a function of time

Okadaic acid (1 µM) was added directly at the start or the chase (zero-time). All other experimental details were as described in the legend to Fig. 2. The results shown are the mean ±SEM of 4 experiments.
(A) Immature βAPP_{751/770}. (B) Mature βAPP_{751/770}. (C) 15 kDa COOH-terminal fragment. Control cells(-●-), okadaic acid-treated cells(-□-).

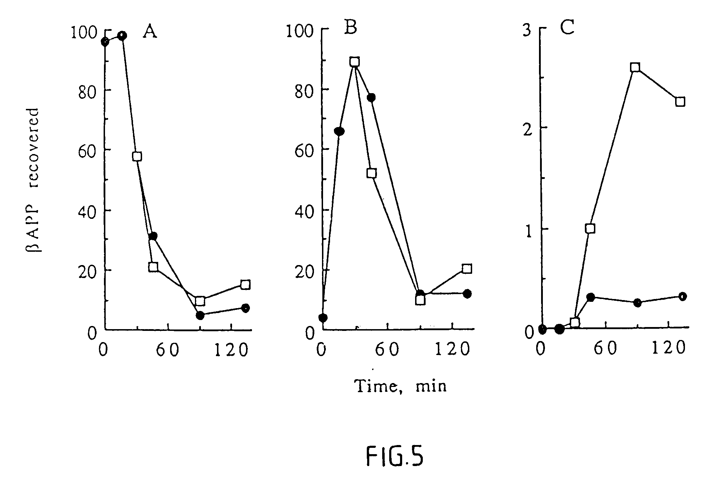

### Figure 5. Regulation by okadaic acid of processing mature βAPP as a function of time

Okadaic acid (1µM) was added at 35 minutes of chase to allow the mature βAPP to approach maximal levels. All other experimental details were as described in the legend to Fig. 2. The experiment shown is representative of two separate experiments. Experimental points are the means of duplicate determination.
(A) Immature βAPP_{751/770}. (B) Mature βAPP_{751/770}. (C) 15 kDa COOH-terminal fragment. Control cells(-●-), okadaic acid-treated cells(-□-).

As reported (Wiedemann et al, supra), βAPP in PC12 cells matures over 45 minutes and is probably processed via a lysosomal pathway. Phorbol esters lead to an apparent stimulation in the rate of this processing. This suggests that protein kinase C (PKC) may target βAPP for internalization and degradation, analogous to its effects in regulating the trafficking of the epidermal growth factor and interleukin-2 receptors. The fact that H7, an inhibitor of PKC, leads to an apparent decrease in the basal rate of processing of βAPP suggests that in the unstimulated cell βAPP may be cleaved after it is phosphorylated by basally active PKC.

The effect of activators of PKC on the levels of the 15kDa and 19 KDa βAPP fragments has been examined in view of the known normal processing of βAPP. It has been reported that the extracellular portion of βAPP is normally secreted after βAPP is cleaved within the B/A4 domain. This means that normal βAPP processing precludes amyloidogenesis and, presumably, cerebral plaque formation. The 15 kDa and 19 kDa C-terminal βAPP fragments have molecular weights consistent with their possibly containing the entire β-amyloid sequence and therefore potentially being amyloidgenic.

The fact that BayK was without effect on βAPP suggests that CaMKII is not involved in βAPP catabolism in applicants' assay System. The effects of ionomycin is believed to be due to effects of this ionophore on calcium gradients across the Golgi membrane.

Okadaic acid, as a potent inhibitor of phosphatases 1 and 2A, increases net phosphorylation for many substrates of numerous protein kinases. Therefore, the effect of okadaic acid on βAPP processing cannot be attributed to any one kinase and is believed to involve phosphorylation of βAPP by kinases distinct from those characterized previously, or phosphorylation of other proteins involved in the regulation of βAPP maturation and processing.

The effects of PDBu and okadaic acid on the 15 and 19 kDa fragments is believed to arise from phosphorylation causing either a more rapid breakdown of the mature βAPP or a slowing down of the catabolism of the 15 and 19 kDa fragments. There is a possibility that the 19 kDa fragment is a phosphorylated form of the 15 kDa fragment.

The following is a list of compounds ("active compounds") which affect or modulate the phosphorylation state of proteins in mammalian cells. Phosphorylation state can be modulated by inhibiting or stimulating the activity of kinases which add a phosphate moiety to proteins, or by inhibiting or stimulating phosphatases which remove a phosphate moiety from proteins.

The following is not meant to be a complete or exhaustive list, but a list of well known examples:

### Kinase stimulators:

Phorbol esters - e.g., 4-alpha-phorbol 12,13-dibutyrate, phorbol 12-myristate 13-acetate (PMA) or (phorbol-12,13-dibutyrate (PDBU or PDBu)
Indolactams - e.g., (-)-7-octylindolactam V
Mezerin
Diacylglycerol
Forskolin
3-(N-acetylamino)-5-(N-decyl-N-methylamino)-benzyl alcohol (ADMB) and
6-(N-decylamino)-4-hydroxymethylindole (DHI)

### Kinase inhibitors:

Staurosporine
Auranofin
W5, W12, W13, W7, H7, H8, H9 or HA1004 (W7 is N-(6-aminohexyl)-5-chloro-1-naphthanesulfamide hydrochloride; H7 is 1-(5-isoquinolinesulfonyl)-2-methylpiperazine dihydrochloride; H8 is N-[2-(methylamino)ethyl]-3-isoquinolinesulfonamide dihydrochloride; H9 is N-(2-aminoethyl)-5-isoquinolinesulfonamide; HA1004 is N(2-guanidinoethyl)-5-isoquinoline-sulfonamide hydrochloride; W5 is N-(6-aminohexyl)-1-napthalensulfonamide hydrochloride; W 12 is N-(4-aminobutyl)-2-naphthalenesulfonamide hydrochloride and W13 is N-(4-aminobutyl)-5-chloro-2-naphthalenesulfonamide hydrochloride)
Sphingosine
Tyrphostin

### Phosphatase inhibitors:

Okadaic acid and derivatives thereof
Calyculin-A
Vanadate

### Phosphatase stimulators:

Somatostatin analogs.

It is to be understood that derivatives of the above stimulators and inhibitors are encompassed by the present invention.

The active compounds for use in the present invention can be administered as a medicament, i.e., a pharmaceutical composition.

The pharmaceutical compositions used in the methods of this invention for administration to animals and humans comprise an active compound in associate with a pharmaceutical carrier or excipient.

The medicament can be in the form of tablets (including lozenges and granules), dragees, capsules, pills, ampoules or suppositories comprising the compound of the invention.

"Medicament" as used herein means physically discrete coherent portions suitable for medical administration. "Medicament in dosage unit form" as used herein means physically discrete coherent units suitable for medical administration, each containing a daily dose or a multiple (up to four times) or a sub-multiple (down to a fortieth) of a daily dose of the active compound of the invention in association with a carrier and/or enclosed within an envelope. Whether the medicament contains a daily dose, or for example, a half, a third or a quarter of a daily dose will depend on whether the medicament is to be administered once or, for example, twice three times or four times a day, respectively.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount, i.e., such that a suitable effective dosage will be consistent with the dosage form employed in single or multiple unit doses. The exact individual dosages, as well as daily dosages, will of course be determined according to standard medical principles under the direction of a physician or veterinarian.

The active compound can also be administered as suspensions, solutions and emulsions of the active compound in aqueous or non-aqueous diluents, syrups, granulates or powders.

Diluents that can be used in pharmaceutical compositions (e.g., granulates) containing the active compound adapted to be formed into tablets, dragees, capsules and pills include the following: (a) fillers and extenders, e.g., starch, sugars, mannitol and silicic acid; (b) binding agents, e.g., carboxymethyl cellulose and other cellulose derivatives, alginates, gelatine and polyvinyl pyrrolidone; (c) moisturizing agents, e.g., glycerol; (d) disintegrating agents, e.g., agar-agar, calcium carbonate and sodium bicarbonate; (e) agents for retarding dissolution, e.g., paraffin; (f) resorption accelerators, e.g., quaternary ammonium compounds; (g) surface active agents, e.g., cetyl alcohol, glycerol monostearate; (h) adsorptive carriers, e.g., kaolin and bentonite; (i) lubricants, e.g., talc, calcium and magnesium stearate and solid polyethyl glycols.

The tablets, dragees, capsules and pills comprising the active compound can have the customary coatings, envelopes and protective matrices, which may contain opacifiers. They can be so constituted that they release the active ingredient only or preferably in a particular part of the intestinal tract, possibly over a period of time. The coatings, envelopes and protective matrices may be made, for example, from polymeric substances or waxes.

The active ingredient can also be made up in microencapsulated form together, with one or several of the above-mentioned diluents.

The diluents to be used in pharmaceutical compositions adapted to be formed into suppositories can, for example, be the usual water-soluble diluents, such as polyethylene glycols and fats (e.g., cocoa oil and high esters, [e.g., C₁₄-alcohol with C₁₆-fatty acid]) or mixtures of these diluents.

The pharmaceutical compositions which are solutions and emulsions can, for example, contain the customary diluents (with, of course, the above-mentioned exclusion of solvents having a molecular weight below 200, except in the presence of a surface-active agent), such as solvents, dissolving agents and emulsifiers. Specific non-limiting examples of such diluents are water, ethyl alcohol, isopropyl alcohol, ethyl carbonate, ethyl acetate, benzyl alcohol, benzyl benzoate, propylene glycol, 1,3-butylene glycol, dimethylformamide, oils (for example, ground nut oil), glycerol, tetrahydrofurfuryl alcohol, polyethylene glycols and fatty acid esters of sorbitol or mixtures thereof.

For parenteral administration, solutions and emulsions should be sterile, e.g., water or arachis oil contained in ampoules and, if appropriate, blood-isotonic.

The pharmaceutical compositions which are suspensions can contain the usual diluents, such as liquid diluents, e.g., water, ethyl alcohol, propylene glycol, surface-active agents (e.g., ethoxylated isostearyl alcohols, polyoxyethylene sorbite and sorbitane esters), microcrystalline cellulose, aluminum methahydroxide, bentonite, agar-agar and tragacanth or mixtures thereof.

The pharmaceutical compositions can also contain coloring agents and preservatives, as well as perfumes and flavoring additions (e.g., peppermint oil and eucalyptus oil) and sweetening agents, (e.g., saccharin and aspartame).

The pharmaceutical compositions will generally contain from 0.5 to 90% of the active ingredient by weight of the total composition.

In addition to the active compound, the pharmaceutical compositions and medicaments can also contain other pharmaceutically active compounds.

Any diluent in the medicaments of the present invention may be any of those mentioned above in relation to the pharmaceutical compositions. Such medicaments may include solvents of molecular weight less than 200 as the sole diluent.

It is envisaged that the active compound will be administered perorally, parenterally (for example, intramuscularly, intraperitoneally, subcutaneously, transdermally or intravenously), rectally or locally, preferably orally or parenterally, especially perlingually, or intravenously.

The dosage rate, e.g., 0.05 to 20 mg/kg of body weight, will be a function of the nature and body weight of the human or animal subject to be treated, the individual reaction of this subject to the treatment, type of formulation in which the active ingredient is administered, the mode in which the administration is carried out and the point in the progress of the disease or interval at which it is to be administered. Thus, it may in some case suffice to use less than a minimum dosage rate, while other cases an upper limit must be exceeded to achieve the desired results. Where larger amounts are administered, it may be advisable to divide these into several individual administrations over the course of the day.

### EXAMPLES

Phorbol 12,13-dibutyrate (PDBu), 4-alpha-phorbol 12,13-dibutyrate (4αPDBu) and phorbol 12-myristate 13-acetate (PMA) were purchased form LC Service Corp., Woburn, Ma. Okadaic acid was purchased from Moana BioProducts, Inc., Honolulu, HI. H7 was purchased form Seikagada Kogyo Co., Japan.

### Example 1

A synthetic peptide corresponding to the cytoplasmic domain of βAPP (βAPP⁶⁴⁵⁻⁶⁹⁴,the numbering of amino acids corresponds to those of human βAPP₆₉₅,) was prepared by the Yale Sequencing Facility. Antibodies were prepared by immunizing rabbits with this synthetic peptide corresponding to βAPP 645-695. Sera were screened by immunoblot analysis of lysates of E.coli expressing a fusion protein including the amino acids 16-695 of human βAPP₆₉₅. Sera which were immunoreactive against the recombinant fusion protein βAPP₆₉₅ were further screened for immunoprecipitating activity in two systems: one system used ³⁵S-labeled βAPP₆₉₅ which was produced by in vitro transcription of βAPP₆₉₅ cDNA followed by in vitro translation of βAPP₆₉₅ mRNA, using rabbit reticulocyte lysate and [³⁵S] methionine; the other system used ³⁵S-labeled βAPP₆₉₅ produced by cells transfected with βAPP ₆₉₅ cDNA and labeled with [³⁵S] methionine. Immunoprecipitation was performed as described in Pang, D.T., Wang, J.K.T., Valtorta, F., Benfenati, F. and Greengard, P., (1988), Proc. Natl. Acad. Sci., U.S.A., 85:762-766. Sera bearing high-efficiency (>90%) immunoprecipitating activity for recombinant βAPP were affinity purified on columns of Sepharose-βAPP645-695 as described in Harlow, E. and Lane, D., Antibodies: A Laboratory Manual (Cold Spring Harbor, N.Y. 1988). The antisera used here recognized three proteins of Mᵣ 115-140 kDa on Western blots of rat cortical homogenate, as well as a band of Mᵣ 93 kDa.

PC cells were grown in Dulbecco's modified Eagle's medium (DMEM, Flow Labs) containing 10% (v/v) fetal calf serum and 5% heat inactivated horse serum.

For metabolic labeling of PC12 cells, the cells were washed twice, removed from the dish by trituration, and the cells were suspended at 6 x 10⁶ cells/ml in methionine-free DMEM, supplemented with 1 mCi/ml of [³⁵S]methionine (1200 Ci/mmol, NEN Products, Boston MA.) and 20 mM Hepes, pH 7.4 for 20 minutes. The cells were then diluted with 5-6 volumes of aerated complete medium (DMEM), containing excess methionine and 20 mM Hepes, pH 7.4, and chased for time periods of 0 to 135 minutes with additions as described. Test substances were added either at the start of the chase period (zero-time) or 35 minutes later. Cell viability, as determined by trypan blue exclusion, was greater than 90% at the start of the experiment and decreased less than 5% over 135 minutes of chase.

For lysis, the cells (0.2 ml) were diluted with 0.4 ml of lysis buffer (2 mM NaN₃, 150 mM NaCl, 100 mM Tris HCl, pH 7.4, containing 1% v/v NP40, 0.5% w/v sodium deoxycholate, 0.1% w/v NaDodSO₄ and 40 units/ml of trasylol) and placed on ice. After 20 minutes, samples were centrifuged at 10,000 x g for 5 minutes, and the supernatants were used for immunoprecipitation. In some experiments, the cells were sometimes lysed with 1% NaDodSO₄, sonicated and boiled and used for immunoprecipitation (Pang, D.T., supra). The results obtained with this procedure were virtually identical to those obtained using the standard procedure.

For immunoprecipitation, the 10,000 x g supernatant was incubated for 1 hour at 4°C with 3 µl of undiluted or affinity-purified rabbit anti-βAPP 645-695 in a total volume of 600 µl. Following the incubation, 75 µl of a 1:1 suspension of Protein A-Sepharose (Pharmacia LKB, Inc., Piscataway, N.J.) in lysis buffer was then added and the incubation was continued for another 30 minutes at 4°C. The insoluble complexes were washed three times with 100 mM Tris HCl, pH 7.4, containing 150 mM NaCl and 2mM NaN₃, resuspended in solubilization buffer (62.5 mM Tris HCl, pH 6.8, 2% SDS, 5% 2-mercaptoethanol, 10% sucrose) and boiled. Proteins were separated by NaDodSO₄-PAGE and detected using quantitative fluorography with 1 M sodium salicylate. Protein bands were quantified either by densitometry of the autoradiograms, or by excising the radioactive bands and subjecting them to liquid scintillation spectometry. βAPP₇₅₁ and βAPP₇₇₀ were the major βAPP isoforms in PC12 cells. Because these isoforms migrated very near each other on PAGE, they were quantified together and denoted βAPP_{751/770}. To quantify βAPP₆₉₅, computational methods were used (Crandell, R., McClellan, M., Arch, S., Doenias, J. and Piper, R., (1987), Anal. Biochem., 167:15). The effects observed for βAPP₆₉₅ were similar to those observed for βAPP_{751/770}. The 19 kDa peptide appeared to behave quantitatively similar to the 15 kDa peptide.

Peptide mapping of immunoprecipitated βAPP or in vitro translated βAPP was performed using s. aureus V8 protease (Miles Co., Elkhart, IN) according to the method of Huttner et al.

### Results

PC12 cells were incubated for 20 minutes in DMEM containing [³⁵S]methionine and subsequently chased for various periods in complete medium. Immunoprecipitation from lysates of such ³⁵S-labeled cells with an antibody prepared against βAPP 645-695 yielded six protein bands of 149, 143, 125, 115, 113 and 106 kDa (Fig. 1a., lanes 1 and 2).

These six proteins were not observed when the immunoprecipitation was carried out in the presence of 100µM βAPP⁶⁴⁵⁻⁶⁹⁴ peptide (Fig. 1A, lane 6). Furthermore, these six proteins exhibited similar peptide maps when digested with V8 protease, and the maps were similar to the maps observed using in vitro transcribed and translated human βAPP ₆₉₅ cDNA (Fig. 1B), strongly evidencing that the immunoprecipitated protein bands are βAPP isoforms.

Changes in the relative amounts of the labeled bands were observed after different durations of the chase period. A possible precursor-product relationship was noted between the doublets of Mᵣ 113/115 and 143/149 kDa, as well as between the bands of Mᵣ 106 and 125 kDa (Figs. 1 and 2). Based on the observations of Wiedemann et al, supra, the proteins of Mᵣ 143/149 and 125 kDa were tentatively identified as follows: Mᵣ 106, 113 and 115 kDa bands, the immature (N-glycosylated) forms of βAPP₆₉₅, βAPP₇₅₁, and βAPP₇₇₀, respectively; Mᵣ 125, 143 and 149 kDa, the mature (N- and O- glycosylated) forms of βAPP₆₉₅, βAPP₇₅₁ and βAPP₇₇₀, respectively. It was previously shown that a population of mature βAPP molecules is rapidly cleaved via a lysosomal mechanism, (Cole, G., Huyhn, T.V. and Saitoh, T., (1989) Neurochem. Res., 14:933) leading to secretion of the N-terminal portion. When 100 µM chloroquine was added to PC12 cells at the beginning of the chase period, the labeled 125/143 and 149 kDa proteins were relatively increased, consistent with the action of chloroquine as an inhibitor of lysosomal acidification and cleavage of the mature βAPP forms.

### Example 2: Regulation of βAPP Processing by Protein Kinase C

When PC12 cells were incubated with 1 µM PDBu during the chase period, no effect was observed on the rate of disappearance of labeled immature βAPP (Fig. 2A). However, the levels of labeled matured forms of βAPP decreased and the levels of labeled peptides of 15 kDa and 19 kDa were increased (Fig. 2B,C). The 15 kDa and 19 kDA peptides were not observed when immunoprecipitation was carried out in the presence of 100µM βAPP⁶⁴⁵⁻⁶⁹⁴ peptide (Fig. 1A, lane 6), consistent with these proteins being COOH-terminal fragments of the mature βAPP. Another phorbol ester, PMA, produced effects similar to those of PDBu, while the inactive PDBu analogue, 4αPDBu was without effect (not shown). The decrease of labeled mature βAPP and concomitant increase of the labeled 15 kDa and 19 kDa peptides evidences a precursor-product relationship between these proteins. Presumably, the 15 kDa and 19 kDa peptides are proteolytic fragments of βAPP which remain associated with the cell after cleavage and secretion of the N-terminal domain. The data evidence that PKC activation increases the rate of processing of mature βAPP, leading to decreased recovery of the mature βAPP and increased recovery of proteolytic fragments.

H7 (100 µM), an inhibitor of several protein kinases including protein kinase C, produced a relative increase in the levels of mature βAPP (Fig. 2B). These observations are consistent with the idea that PKC stimulates the processing of mature βAPP. Moreover, H7, (100 µM) antagonized the effects of PDBu (1µM) in decreasing the levels of labeled mature βAPP and increasing the levels of labeled 15 kDa and 19 kDa peptides (not shown).

A half-maximal effect of PDBu on the levels of labeled mature βAPP was observed at about 17nM, consistent with other effects of PDBu known to be mediated by PKC. In order to examine the effect of activation of PKC on βAPP processing when the levels of mature βAPP neared maximal levels, PDBu (1 µM) was added after 35 minutes of chase. Rapid increases in the levels of labeled 15 kDa and 19 kDa peptides were observed (Fig. 2C), evidencing that PDBu acts to increase the rate of processing of mature βAPP.

### Example 3: Regulation of βAPP Processing by Okadaic acid-sensitive Protein Phosphatases

Addition of okadaic acid ( 1µM) at the start of the chase increased the half-life of labeled immature βAPP, while the amount of labeled mature βAPP fell (Fig. 4A,B). At the same time the labeled 15 kDa and 19 kDa βAPP peptides were increased (Fig. 4C). This evidences that okadaic acid, like PDBu, increases the rate of processing of mature βAPP, but, unlike PDBu, also affects βAPP processing at earlier stages. When okadaic acid (1 µM) was added with PDBu (1 µM) at the start of the chase, levels of the labeled 15 kDa and 19 kDa peptides were greatly increased (Fig. 1A, lane 5). Addition of okadaic acid after 35 minutes of chase, when mature βAPP neared maximal levels, led to a rapid increase in the levels of the labeled 15 kDa and 19 kDa peptides (Fig. 5C). These results evidence that okadaic acid acts to increase the rate of processing of mature βAPP. A half-maximal effect of okadaic acid on the levels of labeled immature and mature βAPP was observed at about 300 nM (not shown).

Applicants have confirmed the prior art premise that βAPP in PC12 cells matures over a period of 45 minutes, and that it is probably processed via a lysosomal pathway. Phorbol esters lead to an apparent stimulation in the rate of processing of mature βAPP and the formation of 15 kDa and 19 kDa peptides. Phorbol esters are believed to have an effect on the rate of catabolism of these peptides. Applicants' results suggest that PKC may target βAPP for internalization and degradation, analogous to its effects in regulating the trafficking of the epidermal growth factor receptor and interleukin-2 receptors (Oltersdorf, T. et al, (1989), JBC, 265:4492-4497). The fact that H7, an inhibitor of PKC, leads to an apparent decrease in the basal rate of processing of βAPP supports the physiological significance of this reaction.

It has been reported that the extracellular portion of βAPP is normally secreted after βAPP is cleaved within the β-amyloid domain (Sisodia, S.S., Kou, E.H., Beyreuther, K., Unterbeck, A. and Price, D.L., (1980), Science, 248:492-495). This means that normal βAPP processing precludes amyloidogenesis and, presumably, cerebral plague formation. The 15 kDa and 19 kDa peptides have apparent molecular weights consistent with their containing the entire β-amyloid sequence and therefore with their potentially being amyloidgenic.

Okadaic acid, a potent inhibitor of phosphatases 1 and 2A, has been shown to increase net phosphorylation for many substrates in various experimental systems. Therefore, the effect of okadaic acid on βAPP processing cannot be attributed to any one kinase and is believed to involve phosphorylation of βAPP by kinases distinct from those characterized previously, or phosphorylation of other proteins involved in the regulation of βAPP maturation and processing.

The observation that PKC and potentially other protein kinases are involved in the regulation of normal and possibly altered processing of βAPP supports the role of abnormal phosphorylation in the amyloidogenesis of Alzheimer's disease.

## Claims (Claims for the following Contracting State(s): AT, BE, CH, DE, DK, FR, GB, GR, IT, LI, LU, NL, SE)

1. Use of at least one kinase modulator or phosphatase modulator which increases or decreases the rate of proteolytic processing of proteins found in intracellular neurofibrillary tangles and extracellular amyloid plaques for the preparation of a pharmaceutical composition for the treatment of amyloidosis associated with Alzheimer's disease.

2. The use according to claim 1, wherein said proteins are selected from the group consisting of βA4 amyloid precursor protein, tau or a neurofilament protein.

3. The use according to claim 1 or 2, wherein said modulator is a kinase stimulator selected from the group consisting of a phorbol ester, an indolactam, mezerin, diacylglycerol or forskolin.

4. The use according to claim 3, wherein said kinase stimulator is a phorbol ester selected from the group consisting of 4-alpha-phorbol 12,13-dibutyrate, phorbol-12,13-dibutyrate and phorbol 12-myristate 13-acetate.

5. The use according to claim 3, wherein said kinase stimulator is (-)-7-octylindolactam V.

6. The use according to claim 1 or 2, wherein said modulator is a kinase inhibitor selected from the group consisting of staurosporine, auranofin, W5, W12, W13, W7, H7, H8, H9, HA1004, sphingosine and tyrphostin.

7. The use according to claim 1, wherein said modulator is a phosphatase stimulator, including a somatostatin analog.

8. The use according to claim 1 or 2, wherein said phosphatase modulator is at least one inhibitor of protein phosphatase, said inhibitor decreasing the proteolytic processing of proteins found in intracellular neurofibrillary tangles and extracellular amyloid plaques.

9. The use according to claim 8, wherein said inhibitor of protein phosphatase is okadaic acid, a derivative of okadaic acid, calyculin-A or vanadate.

10. An in vitro method of screening for an agent that modulates amyloid formation comprising contacting mammalian cells with an agent suspected of being capable of modulating the phosphorylation of proteins and detecting any alterations in the degradation of the amyloid precursor protein
comprising the steps of:
(a) providing mammalian cells or tissue sections in culture;
(b) radioactively labeling proteins produced by the mammalian cells during anabolism; then
(c) allowing the mammalian cells to continue metabolizing in a suitable, label-free media;
(d) contacting the mammalian cells at the start of or during step (c) with an agent suspected of being capable of modulating phosphorylation of proteins that occurs during cell metabolism;
(e) lysing the mammlian cells;
(f) immunoprecipitating the labeled βAPP fragments moieties with an antibody against βAPP; and
(g) comparing the immunoprecipitated βAPP or βAPP fragments to standard βAPP or βAPP fragments to detect changes in βAPP degradation and β-amyloid production.

11. Use of an agent that modulates phosphorylation of proteins for the preparation of a diagnostic composition for screening for an agent that modulates amyloid formation comprising administering said diagnostic composition to a normal or transgenic whole animal and detecting neurodegenerative changes in the brain of the animal.

## Claims (Claims for the following Contracting State(s): ES)

1. A method for preparing a pharmaceutical composition for the treatment of amyloidosis associated with Alzheimer's disease which comprises combining at lease one kinase modulator or phosphatase modulator which increases or decreases the rate of proteolytic processing of proteins found in intracellular neurofibrillary tangles and extracellular amyloid plagues with a physiologically acceptable carrier.

2. The method according to claim 1, wherein said proteins are selected from the group consisting of βA4 amyloid precursor protein, tau or a neurofilament protein.

3. The method according to claim 1 or 2, wherein said modulator is a kinase stimulator selected from the group consisting of a phorbol ester, an indolactam, mezerin, diacylglycerol or forskolin.

4. The method according to claim 3, wherein said kinase stimulator is a phorbol ester selected from the group consisting of 4-alpha-phorbol 12,13-dibutyrate, phorbol-12,13-dibutyrate and phorbol 12-myristate 13-acetate.

5. The method according to claim 3, wherein said kinase stimulator is (-)-7-octylindolactam V.

6. The method according to claim 1 or 2, wherein said modulator is a kinase inhibitor selected from the group consizing of staurosporine, auranofin, W5, W12, W13, W7, H7, H8, H9, HA1004, sphingosine and tyrphostin.

7. The method according to claim 1, wherein said modulator is a phosphatase stimulator, including a somatostatin analog.

8. The method according to claim 1 or 2, wherein said phosphatase modulator is at least one inhibitor of protein phosphatase, said inhibitor decreasing the proteolytic processing of proteins found in intracellular neurofibrillary tangles and extracellular amyloid plaques.

9. The method according to claim 8, wherein said inhibitor of protein phosphatase is okadaic acid, a derivative of okadaic acid, calyculin-A or vanadate.

10. An in vitro method of screening for an agent that modulates amyloid formation comprising contacting mammalian cells with an agent suspected of being capable of modulating the phosphorylation of proteins and detecting any alterations in the degradation of the amyloid precursor protein, said method
comprising the steps of:
(a) providing mammalian cells or tissue sections in culture;
(b) radioactively labeling proteins produced by the mammalian cells during anabolism; then
(c) allowing the mammalian cells to continue metabolizing in a suitable, label-free media;
(d) contacting the mammalian cells at the start of or during step (c) with an agent suspected of being capable of modulating phosphorylation of proteins that occurs during cell metabolism;
(e) lysing the mammlian cells;
(f) immunoprecipitating the labeled βAPP fragments moieties with an antibody against βAPP; and
(g) comparing the immunoprecipitated βAPP or βAPP fragments to standard βAPP or βAPP fragments to detect changes in APP degradation and β-amyloid production.

11. A method of preparing a diagnostic composition for screening for an agent that modulates amyloid formation comprising administering said diagnostic composition to a normal or transgenic whole animal and detecting neuro-degenerative changes in the brain of the animal which comprises combining an agent that
modulates phosphorylation of proteins with a suitable carrier.

## Patentansprüche (Patentansprüche für folgende(n) Vertragsstaat(en): AT, BE, CH, DE, DK, FR, GB, GR, IT, LI, LU, NL, SE)

1. Verwendung mindestens eines Kinasemodulators oder Phosphatasemodulators, der die Rate der proteolytischen Prozessierung von Proteinen erhöht oder erniedrigt, die sich in intrazellulären Alzheimer-Fibrillen ("neurofibrillary tangles") und extrazellulären Amyloidplaques befinden, zur Herstellung eines Arzneimittels für die Behandlung der Amyloidosis im Zusammenhang mit der Alzheimer-Krankheit.

2. Verwendung nach Anspruch 1, wobei die Proteine ausgewählt sind aus βA4-Amyloidvorläuferprotein, Tau oder einem Neurofilamentprotein.

3. Verwendung nach Anspruch 1 oder 2, wobei der Modulator ein Kinasestimulator ist, ausgewählt aus einem Phorbolester, einem Indolactam, Mezerin, Diacylglycerin oder Forskolin.

4. Verwendung nach Anspruch 3, wobei der Kinasestimulator ein Phorbolester ist, ausgewählt aus 4-α-Phorbol-12,13-dibutyrat, Phorbol-12,13-dibutyrat und Phorbol-12-myristat-13-acetat.

5. Verwendung nach Anspruch 3, wobei der Kinasestimulator (-)-7-Octylindolactam V ist.

6. Verwendung nach Anspruch 1 oder 2, wobei der Modulator ein Kinaseinhibitor ist, ausgewählt aus Staurosporin, Auranofin, W5, W12, W13, W7, H7, H8, H9, HA1004, Sphingosin und Tyrphostin.

7. Verwendung nach Anspruch 1, wobei der Modulator ein Phosphatasestimulator ist, einschließlich eines Somatostatinanalogs.

8. Verwendung nach Anspruch 1 oder 2, wobei der Phosphatasemodulator mindestens ein Inhibitor von Proteinphosphatase ist und der Inhibitor die proteolytische Prozessierung von Proteinen verringert, die sich in intrazellulären Alzheimer-Fibrillen und in extrazellulären Amyloidplaques befinden.

9. Verwendung nach Anspruch 8, wobei der Inhibitor der Proteinphosphatase Okadasäure, ein Derivat von Okadasäure, Calyculin-A oder Vanadat ist.

10. In vitro-Verfahren zur Durchmusterung auf einen Wirkstoff, der die Amyloiderzeugung moduliert, umfassend das Inkontaktbringen von Säugerzellen mit einem Wirkstoff, von dem angenommen wird, daß er zur Modulierung der Phosphorylierung von Proteinen fähig ist, und den Nachweis von Veränderungen im Abbau des Amyloidvorläuferproteins, umfassend die Schritte:
(a) Bereitstellen von Säugerzellen oder Gewebsschnitten in Kultur;
(b) radioaktive Markierung von Proteinen, die von der Säugerzelle während ihres Anabolismus produziert werden;
(c) Ermöglichung für die Säugerzellen, die Metabolisierung in einem geeigneten, markerfreien Medium fortzusetzen;
(d) Inkontaktbringen der Säugerzellen zu Beginn oder während des Schrittes (c) mit einem Wirkstoff, von dem angenommen wird, daß er zur Modulation der Phosphorylierung von Proteinen fähig ist, die während des Zellmetabolismus erfolgt;
(e) Lysieren der Säugerzellen;
(f) Immunpräzipitation der markierten βAPP-Fragmenteinheiten mit einem Antikörper gegen βAPP; und
(g) Vergleich der immunpräzipitierten βAPP oder βAPP-Fragmente mit Standard-βAPP oder -βAPP-Fragmenten, um Veränderungen im βAPP-Abbau und in der β-Amyloidproduktion nachzuweisen.

11. Verwendung eines Wirkstoffs, der die Phosphorylierung von Proteinen moduliert, zur Herstellung einer diagnostischen Zusammensetzung für die Durchmusterung auf einen Wirkstoff, der die Amyloiderzeugung moduliert, umfassend die Verabreichung der diagnostischen Zusammensetzung an ein normales oder transgenes ganzes Tier und der Nachweis von neurodegenerativen Veränderungen im Gehirn des Tieres.

## Patentansprüche (Patentansprüche für folgende(n) Vertragsstaat(en): ES)

1. Verfahren zur Herstellung eines Arzneimittels für die Behandlung von Amyloidosis im Zusammenhang mit der Alzheimer-Krankheit, umfassend das Kombinieren mindestens eines Kinasemodulators oder eines Phosphatasemodulators, der die Rate der proteolytischen Prozessierung von Proteinen erhöht oder erniedrigt, die sich in intrazellulären Alzheimer-Fibrillen ("neurofibrillary tangles") und extrazellulären Amyloidplaques befinden, mit einem physiologisch verträglichen Träger.

2. Verfahren nach Anspruch 1, wobei die Proteine ausgewählt sind aus βA4-Amyloidvorläuferprotein, Tau oder einem Neurofilamentprotein.

3. Verfahren nach Anspruch 1 oder 2, wobei der Modulator ein Kinasestimulator ist, ausgewählt aus einem Phorbolester, einem Indolactam, Mezerin, Diacylglycerin oder Forskolin.

4. Verfahren nach Anspruch 3, wobei der Kinasestimulator ein Phorbolester ist, ausgewählt aus 4-α-Phorbol-12,13-dibutyrat, Phorbol-12,13-dibutyrat und Phorbol-12-myristat-13-acetat.

5. Verfahren nach Anspruch 3, wobei der Kinasestimulator (-)-7-Octylindolactam V ist.

6. Verfahren nach Anspruch 1 oder 2, wobei der Modulator ein Kinaseinhibitor ist, ausgewählt aus Staurosporin, Auranofin, W5, W12, W13, W7, H7, H8, H9, HA1004, Sphingosin und Tyrphostin.

7. Verfahren nach Anspruch 1, wobei der Modulator ein Phosphatasestimulator ist, einschließlich eines Somatostatinanalogs.

8. Verfahren nach Anspruch 1 oder 2, wobei der Phosphatasemodulator mindestens ein Inhibitor von Proteinphosphatase ist und der Inhibitor die proteolytische Prozessierung von Proteinen verringert, die sich in intrazellulären Alzheimer-Fibrillen und in extrazellulären Amyloidplaques befinden.

9. Verfahren nach Anspruch 8, wobei der Inhibitor der Proteinphosphatase Okadasäure, ein Derivat von Okadasäure, Calyculin-A oder Vanadat ist.

10. In vitro-Verfahren zur Durchmusterung auf einen Wirkstoff, der die Amyloiderzeugung moduliert, umfassend das Inkontaktbringen von Säugerzellen mit einem Wirkstoff, von dem angenommen wird, daß er zur Modulierung der Phosphorylierung eines Proteins fähig ist, und den Nachweis von Veränderungen im Abbau des Amyloidvorläuferproteins, umfassend die Schritte:
(a) Bereitstellen von Säugerzellen oder Gewebsschnitten in Kultur;
(b) radioaktive Markierung von Proteinen, die von der Säugerzelle während ihres Anabolismus produziert werden;
(c) Ermöglichung für die Säugerzellen, die Metabolisierung in einem geeigneten, markerfreien Medium fortzusetzen;
(d) Inkontaktbringen der Säugerzellen zu Beginn oder während des Schrittes (c) mit einem Wirkstoff, von dem angenommen wird, daß er zur Modulation der Phosphorylierung von Proteinen fähig ist, die während des Zellmetabolismus erfolgt;
(e) Lysieren der Säugerzellen;
(f) Immunpräzipitation der markierten βAPP-Fragmenteinheiten mit einem Antikörper gegen βAPP; und
(g) Vergleich der immunpräzipitierten βAPP oder βAPP-Fragmente mit Standard-βAPP oder -βAPP-Fragmenten, um Veränderungen im βAPP-Abbau und in der β-Amyloidproduktion nachzuweisen.

11. Verfahren zur Herstellung einer diagnostischen Zusammensetzung für die Durchmusterung auf einen Wirkstoff, der die Amyloiderzeugung moduliert, umfassend die Verabreichung der diagnostischen Zusammensetzung an ein normales oder transgenes ganzes Tier und den Nachweis von neurodegenerativen Veränderungen im Gehirn des Tieres, umfassend die Kombination eines Wirkstoffs, der die Phosphorylierung von Proteinen moduliert, mit einem geeigneten Träger.

## Revendications (Revendications pour l'(les) Etat(s) contractant(s) suivant(s): AT, BE, CH, DE, DK, FR, GB, GR, IT, LI, LU, NL, SE)

1. Utilisation d'au moins un modulateur de kinase ou modulateur de phosphatase qui augmente ou diminue la vitesse de traitement protéolytique des protéines trouvées dans les enchevêtrements neurofibrillaires intracellulaires et plaques amyloïdes extracellulaires pour la préparation d'une composition pharmaceutique dans le traitement de l'amyloïdose associée à la maladie d'Alzheimer.

2. Utilisation suivant la revendication 1, dans laquelle les protéines précitées sont choisies dans le groupe comprenant la protéine précurseur amyloïde βA4, la protéine tau ou une protéine neurofilamentaire.

3. Utilisation suivant l'une ou l'autre des revendications 1 et 2, dans laquelle le modulateur est unstimulateurde kinase choisi dans le groupe comprenant un ester de phorbol, un indolactame, la mézérine, le diacylglycérol ou la forskoline.

4. Utilisation suivant la revendication 3, dans laquelle le stimulateur de kinase est un ester de phorbol choisi dans le groupe comprenant le 12,13-dibutyrate de 4-α-phorbol, le 12,13-dibutyrate de phorbol et le 12-myristate 13-acétate de phorbol.

5. Utilisation suivant la revendication 3, dans laquelle le stimulateur de kinase est le (-)-7-octylindolactame V.

6. Utilisation suivant l'une ou l'autre des revendications 1 et 2, dans laquelle le modulateur est un inhibiteur de kinase choisi dans le groupe comprenant la staurosporine, l'auranofine, le W5, le W12, le W13, le W7, le H7, le H8, le H9, le HA1004, la sphingosine et la tyrphostine.

7. Utilisation suivant la revendication 1, dans laquelle le modulateur est un stimulateur de phosphatase, notamment un analogue de somatostatine.

8. Utilisation suivant l'une ou l'autre des revendications 1 et 2, dans laquelle le modulateur de phosphatase est au moins un inhibiteur de protéine phosphatase, ledit inhibiteur diminuant le traitement protéolytique des protéines trouvées dans les enchevêtrements neurofibrillaires intracellulaires et les plaques amyloïdes extracellulaires.

9. Utilisation suivant la revendication 8, dans laquelle l'inhibiteur de protéine phosphatase est l'acide okadaïque, un dérivé d'acide okadaïque, la calyculine-A ou le vanadate.

10. Méthode de dépistage in vitro d'un agent qui module la formation d'amyloïde comprenant la mise en contact de cellules mammifères avec un agent suspecté de moduler la phosphorylation de protéine et la détection des éventuelles modifications dans la dégradation de la protéine précurseur amyloïde, comprenant les étapes suivantes :
(a) la formation de cellules mammifères ou de sections tissulaires en culture;
(b) le marquage radioactif des protéines produites par les cellules mammifères en cours d'anabolisme; ensuite
(c) la poursuite de la métabolisation des cellules mammifères dans un milieu sans marquage, approprié;
(d) la mise en contact des cellules mammifères au commencement ou pendant l'étape (c) avec un agent suspecté de pouvoir moduler le phosphorylation des protéines qui se produit au cours du métabolisme cellulaire;
(e) la lyse des cellules mammifères;
(f) l'immunoprécipitation des fragments de βAPP marqués avec un anticorps contre la βAPP; et
(g) la comparaison de la βAPP ou des fragments de βAPP immunoprécipités avec de la βAPP ou des fragments de βAPP standards pour détecter les modifications dans la dégradation de la βAPP et la production de β-amyloïde.

11. Utilisation d'un agent qui module la phosphorylation de protéines pour la préparation d'une composition de diagnostic pour le dépistage d'un agent qui module la formation d'amyloïde comprenant l'administration de ladite composition de diagnostic à un animal entier normal ou transgénique et la détection des changements neurodégénératifs dans le cerveau de l'animal.

## Revendications (Revendications pour l'(les) Etat(s) contractant(s) suivant(s): ES)

1. Procédé de préparation d'une composition pharmaceutique pour le traitement de l'amyloïdose associée à la maladie d'Alzheimer, qui comprend la combinaison d'au moins un modulateur de kinase ou modulateur de phosphatase qui augmente ou diminue la vitesse de traitement protéolytique des protéines trouvées dans les enchevêtrements neurofibrillaires intracellulaires et les plaques amyloïdes extracellulaires avec un support physiologiquement acceptable.

2. Procédé suivant la revendication 1, dans lequel les protéines précitées sont choisies dans le groupe comprenant la protéine précurseur amyloïde βA4, la protéine tau ou une protéine neurofilamentaire.

3. Procédé suivant l'une ou l'autre des revendications 1 et 2, dans lequel le modulateur est un stimulateur de kinase choisi dans le groupe comprenant un ester de phorbol, un indolactame, la mézérine, le diacylglycérol ou la forskoline.

4. Procédé suivant la revendication 3, dans lequel le stimulateur de kinase est un ester de phorbol choisi dans le groupe comprenant le 12,13-dibutyrate de 4-α-phorbol, le 12,13-dibutyrate de phorbol et le 12-myristate 13-acétate de phorbol.

5. Procédé suivant la revendication 3, dans lequel le stimulateur de kinase est le (-)-7-octylindolactame V.

6. Procédé suivant l'une ou l'autre des revendications 1 et 2, dans lequel le modulateur est un inhibiteur de kinase choisi dans le groupe comprenant la staurosporine, l'auranofine, le W5, le W12, le W13, le W7, le H7, le H8, le H9, le HA1004, la sphingosine et la tyrphostine.

7. Procédé suivant la revendication 1, dans lequel le modulateur est un stimulateur de phosphatase, notamment un analogue de somatostatine.

8. Procédé suivant l'une ou l'autre des revendications 1 et 2, dans lequel le modulateur de phosphatase est au moins un inhibiteur de protéine phosphatase, ledit inhibiteur diminuant le traitement protéolytique des protéines trouvées dans les enchevêtrements neurofibrillaires intracellulaires et les plaques amyloïdes extracellulaires.

9. Procédé suivant la revendication 8, dans lequel l'inhibiteur de protéine phosphatase est l'acide okadaïque, un dérivé d'acide okadaïque, la calyculine-A ou le vanadate.

10. Méthode de dépistage in vitro d'un agent qui module la formation d'amyloïde comprenant la mise en contact de cellules mammifères avec un agent suspecté de moduler la phosphorylation de protéine et la détection des éventuelles modifications dans la dégradation de la protéine précurseur amyloïde, ladite méthode comprenant les étapes suivantes :
(a) la formation de cellules mammifères ou de sections tissulaires en culture;
(b) le marquage radioactif des protéines produites par les cellules mammifères en cours d'anabolisme; ensuite
(c) la poursuite de la métabolisation des cellules mammifères dans un milieu sans marquage, approprié;
(d) la mise en contact des cellules mammifères au commencement ou pendant l'étape (c) avec un agent suspecté de pouvoir moduler le phosphorylation des protéines qui se produit au cours du métabolisme cellulaire;
(e) la lyse des cellules mammifères;
(f) l'immunoprécipitation des fragments de βAPP marqués avec un anticorps contre la βAPP; et
(g) la comparaison de la βAPP ou des fragments de βAPP immunoprécipités avec de la βAPP ou des fragments de βAPP standards pour détecter les modifications dans la dégradation de la βAPP et la production de β-amyloïde.

11. Procédé de préparation d'une composition de diagnostic pour le dépistage d'un agent qui module la formulation d'amyloïde comprenant l'administration de ladite composition de diagnostic à un animal entier normal ou transgénique et la détection des changements neurodégénératifs dans le cerveau de l'animal qui comprend la combinaison d'un agent qui module la phosphorylation de protéines avec un support approprié.
